# EUROPEAN PATENT SPECIFICATION

(11) **EP 2 829 321 B1**
(45) Date of publication and mention of the grant of the patent: **18.11.2026**
(21) Application number: 13764774.9
(22) Date of filing: 22.03.2013
(51) Int. Cl.: B01J 23/889, B01J 23/26, B01J 23/34, C01B 3/02, C07C 1/02, C07C 9/04, C07C 29/153, C07C 31/04, C07B 61/00

(54) **CATALYST FOR MANUFACTURING THERMOCHEMICAL FUEL, AND METHOD FOR MANUFACTURING THERMOCHEMICAL FUEL**
KATALYSATOR ZUR HERSTELLUNG EINES THERMOCHEMISCHEN KRAFTSTOFFES UND VERFAHREN ZUR HERSTELLUNG EINES THERMOCHEMISCHEN KRAFTSTOFFES
CATALYSEUR DESTINÉ À LA PRODUCTION DE COMBUSTIBLE THERMOCHIMIQUE ET PROCÉDÉ DE PRODUCTION DE COMBUSTIBLE THERMOCHIMIQUE

(30) Priority: 23.03.2012 US 201261615122 P; 31.08.2012 US 201213600948
(43) Date of publication of application: 28.01.2015
(73) Proprietor: Japan Science and Technology Agency, Kawaguchi-shi Saitama 332-0012 (JP); California Institute of Technology, Pasadena, CA 91125 (US)
(72) Inventor: YAMAZAKI Yoshihiro, Pasadena, California 91125 (US); HAILE Sossina M., Pasadena, California 91125 (US); YANG Chih-Kai, Pasadena, California 91125 (US)
(74) Representative: Plasseraud IP
(86) International application number: PCT/JP2013/058431
(87) International publication number: WO 2013/141385

(56) References cited:
- CN-A- 1 302 781
- JP-A- 2004 269 296
- US-A1- 2009 107 044
- US-A1- 2011 300 064
- NALBANDIAN L ET AL: "La1-xSrxMO3 (M = Mn, Fe) perovskites as materials for thermochemical hydrogen production in conventional and membrane reactors", INTERNATIONAL JOURNAL OF HYDROGEN ENERGY, ELSEVIER SCIENCE PUBLISHERS B.V., BARKING, GB, vol. 34, no. 17, September 2009 (2009-09-01), pages 7162 - 7172, XP026459630, ISSN: 0360-3199, [retrieved on 20090724]
- LORI NALBANDIAN ET AL: "LaSrMFeOperovskites as oxygen-carrier materials for chemical-looping reforming", INTERNATIONAL JOURNAL OF HYDROGEN ENERGY, ELSEVIER SCIENCE PUBLISHERS B.V., BARKING, GB, vol. 36, no. 11, 28 February 2011 (2011-02-28), pages 6657 - 6670, XP028384848, ISSN: 0360-3199, [retrieved on 20110308], DOI: 10.1016/J.IJHYDENE.2011.02.146
- MORI MASAHI: "Effect of B-site doing on thermal cycle shrinkage for La0.8Sr0.2Mn1-xMxO3+delta perovskites (M=Mg, Al, Ti, Mn, Fe, Co, Ni; 0<=x<=0.1)", SOLID STATE IONICS, NORTH HOLLAND PUB. COMPANY. AMSTERDAM; NL, NL, vol. 174, no. 1-4, 29 October 2004 (2004-10-29), pages 1 - 8, XP004663542, ISSN: 0167-2738, DOI: 10.1016/J.SSI.2004.07.024
- DATABASE WPI Week 201152, Derwent World Patents Index; AN 2011-G22197, XP002751408
- LEION H ET AL: "Use of CaMn0.875Ti0.125O3 as Oxygen Carrier in Chemical-Looping with Oxygen Uncoupling", ENERGY & FUELS, AMERICAN CHEMICAL SOCIETY, WASHINGTON, DC, US, vol. 23, 27 August 2009 (2009-08-27), pages 5276 - 5283, XP002604307, ISSN: 0887-0624, DOI: 10.1021/EF900444D
- MAGNUS RYDN ET AL: "CaMnTiOas oxygen carrier for chemical-looping combustion with oxygen uncoupling (CLOU)Experiments in a continuously operating fluidized-bed reactor system", INTERNATIONAL JOURNAL OF GREENHOUSE GAS CONTROL, ELSEVIER LTD, GB, vol. 5, no. 2, August 2010 (2010-08-01), pages 356 - 366, XP028153029, ISSN: 1750-5836, [retrieved on 20100806], DOI: 10.1016/J.IJGGC.2010.08.004
- ALAN THURSFIELD ET AL: "Chemical looping and oxygen permeable ceramic membranes for hydrogen production - a review", ENERGY & ENVIRONMENTAL SCIENCE, vol. 5, no. 6, 1 March 2012 (2012-03-01), UK, pages 7421, XP055230169, ISSN: 1754-5692, DOI: 10.1039/c2ee03470k
- EVDOU, A. ET AL.: "La(1-x)SrxMnO3-delta perovskites as redox materials for the production of high purity hydrogen", INT. J. HYDROGEN ENERGY, vol. 33, 2008, pages 5554 - 5562, XP055169034

## Description

### [Technical Field]

The present invention relates to catalysts for thermochemical fuel production, and a method of producing fuel using thermochemical fuel production.

Priority is claimed on U.S. Provisional Application Ser. No. 61/615,122, filed March 23, 2012, and U.S. Application No. 13/600,948, filed August 31, 2012.

### [Background Art]

A thermochemical fuel generating method is a technology of producing a chemical fuel from thermal energy that may be obtained from sunlight and the like, and of storing the thermal energy as a chemical fuel. The fuel is produced by a two-step thermal cycle (thermochemical cycle) which includes a step performed under a high temperature (first temperature) and a step performed under a low temperature (second temperature).. In practice, when a catalyst oxide is reduced at a high temperature, and carbon dioxide that is a raw material gas, water vapor, and the like are introduced to the reduced catalyst oxide, the catalyst oxide absorbs oxygen from a raw material, and thus fuels such as a mixed gas, methane, hydrocarbon, alcohol, and hydrogen may be produced.

As oxide-based catalysts, mainly, ZnO-Zn, Fe₂O₃-FeO, CeO₂-Ce₂O₃, CeO₂ having a nonstoichiometric composition, mixtures thereof, a partially substituted oxide, and the like have been reported. Water vapor modification of methane (CH₄+H₂O→6H₂+CO) using LaSrMnO₃-based perovskite oxide has been reported, but this is completely different from thermochemical fuel generation (H₂O→H₂+1/2O₂, or CO₂→CO+1/2O₂) using water or carbon dioxide, and thermochemical fuel production using a perovskite oxide AXO₃ has not been reported until now.

Among fuels that may be generated by the thermochemical fuel generation method, for example, hydrogen is a clean energy source that generates only water after combustion, and thus hydrogen is expected as renewable energy.

When hydrogen is produced by directly decomposing water (H₂O→H₂+1/2O₂), a high temperature of several thousands of °C is necessary, but when using the thermochemical fuel generation method, hydrogen may be produced by decomposing water by a thermal cycle of two-step temperature of relatively low temperature (for example, refer to Patent Document 1).

In the two-step thermal cycle (thermochemical cycle) which includes a step performed under a high temperature (first temperature) and a step performed under a low temperature (second temperature), with respect to the high temperature heating, a technology using solar energy is known (for example, Patent Document 2).

Solar energy is, by far, the most abundant renewable energy source. To fully take advantage of this vast energy source, it must be efficiently stored in a stable form on a massive scale. To store sunlight into chemical forms, solar thermochemical fuel production using nonstoichiometric oxide has been researched. It is a two-step thermochemical cycle that drives redox reaction between the oxide and gas species. The oxide is reduced at a high temperature at which oxygen is released from the oxide. Then, at a lower temperature at which carbon dioxide and/or water vapor are introduced, the oxide strips oxygen atoms from the introduced gas. As a result, syngas, methane, and hydrogen fuels are produced. The thermodynamic efficiency is calculated to be 15 to 75% depending on the oxide systems including ZnO-Zn, Fe₂O₃-FeO, CeO₂-Ce₂O₃, nonstoichiometric CeO₂ systems, and some combinations between them. Other systems have been largely unexplored. A record solar-fuel conversion efficiency is 0.8% in a solar-thermochemical cycle of 800 to 1,630°C using undoped ceria, with 1.3 to 1.5 liters of carbon monoxide and hydrogen production.

As catalyst oxides that are used in the thermochemical fuel generation method, cerium oxide (ceria) is known (Non-Patent Document 1). In the solar thermochemical efficiency experiment using undoped ceria, the solar reactor lost energy of 50% or less as heat, specifically above 1,250°C, and energy of 40% or less as solar re-reflection from the aperture. Thus, the large improvement in solar-fuel conversion efficiency can be anticipated. To address this issue, a mechanical engineering approach and a materials science approach are possible. A heat recovery system might also be integrated. The challenge in this route is how to choose an appropriate oxide structure as well as materials chemistry process from millions of candidate oxides that might show the desired properties. Combinatorial synthesis might be very useful to make candidate oxides, but a rapid way to check fuel productivity at high temperatures is required.

### [Prior Art Document]

### [Patent Document]

[Patent Document 1] Japanese Unexamined Patent Application, First Publication No. 2004-269296
[Patent Document 2] Japanese Unexamined Patent Application, First Publication No. 2009-263165
[Patent Document 3] Specification of US Patent Application Publication No. 2009/0107044

### [Disclosure of the Invention]

### [Problem that the Invention is to solve]

An object of the document is to provide a catalyst, which is formed from a perovskite oxide, for thermochemical fuel production, and a method of producing fuel using thermochemical fuel production that is capable of allowing a fuel to be produced in a thermochemical manner.

### [Means for Solving the Problem]

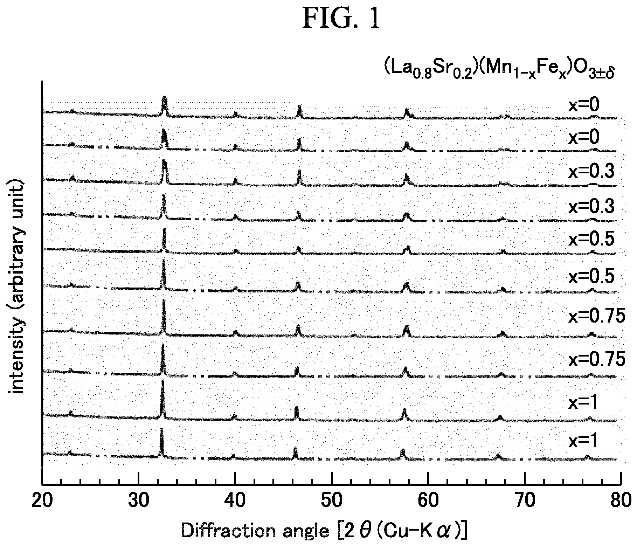

The invention is defined by the claims. The document proposes catalysts for thermochemical fuel production, particularly, bio-inspired catalytic perovskite for solar thermochemical water splitting. Natural photosynthesis, specifically, water oxidation is catalyzed in the Mn₄CaO₅ cluster that possesses the cubic-like frame with a projection. It is anticipated that the artificial water splitting might occur in a similar cubic-like structure containing manganese elements. This hypothesis led to the thermochemical water splitting experiment using manganese-based perovskite (a cubic-like structure, a structure shown on the right of graph in FIG. 1). The perovskite splits water and produces hydrogen with the amount exceeding the amount of hydrogen the ceria produces . As far as the present inventors know, this is the first demonstration of thermochemical water splitting using nonstoichiometric perovskite oxides. The Sr-doped LaMnO₃ perovskites were utilized in the steam reforming of methane, but no thermochemical water splitting has been demonstrated in perovskite oxides. The black color of the perovskite, compared with the white color of ceria, would be beneficial in efficient solar absorbance, thus making possible efficient solar-fuel conversion.

The two-step thermochemical cycle reaction, which uses the perovskite oxide as catalyst for hydrogen production as an example of the catalyst for the thermochemical fuel production, includes the following two steps of an oxygen releasing reaction and a hydrogen generating reaction.

[Oxygen Releasing Reaction (High-Temperature Reduction Reaction)] AXO_{3±δ} → AXO_{3±δ-α} + (α/2)O₂

[Hydrogen Generating Reaction (Low-Temperature Oxidation Reaction)] AXO_{3±δ-α} + αH₂O → AXO_{3±δ} + αH₂

In addition, the entire reaction is represented as follows.

[Entire Reaction] αH₂O → αH₂ + (α/2)O₂

In addition, the two-step thermochemical cycle reaction, which uses the perovskite oxide as a catalyst for thermochemical methane production as an example of the catalyst for the thermochemical fuel production, includes the following two steps of an oxygen releasing reaction and a methane generating reaction.

[Oxygen Releasing Reaction (High-Temperature Reduction Reaction)] AXO_{3±δ} → AXO_{3±δ-α} + (α/2)O₂

[Methane Generating Reaction (Low-Temperature Oxidation Reaction)] AXO_{3±δ-α} + (α/4)CO₂ + (α/2)H₂O → AXO_{3±δ} + (α/4)CH₄

In addition, the entire reaction is represented as follows.

[Entire Reaction] (α/4)CO₂ + (α/2)H₂O → (α/4)CH₄ + (α/2)O₂

In addition, the two-step thermochemical cycle reaction, which uses the perovskite oxide as a catalyst for thermochemical methanol production as an example of the catalyst for the thermochemical fuel production, includes the following two steps of an oxygen releasing reaction and a methanol generating reaction.

[Oxygen Releasing Reaction (High-Temperature Reduction Reaction)] AXO_{3±δ} → AXO_{3±δ-α} + (α/2)O₂

[Methanol Generating Reaction (Low-Temperature Oxidation Reaction)] AXO_{3±δ-α} + (α/3)CO₂ + (2 α/3)H₂O → AXO_{3±δ} + (δ/3)CH₃OH

In addition, the entire reaction is represented as follows.

[Entire Reaction] (α/3)CO₂ + (2α/3)H₂O → (α/3)CH₃OH + (α/2)O₂

**In** addition, the two-step thermochemical cycle reaction, which uses the perovskite oxide as a catalyst for thermochemical methanol production as an example of the catalyst for the thermochemical fuel production, includes the following two steps of an oxygen releasing reaction and a carbon monoxide generating reaction.

[Oxygen Releasing Reaction (High-Temperature Reduction Reaction)] AXO_{3±δ} → AXO_{3±δ-α} + (α/2)O₂

[Carbon Monoxide Generating Reaction (Low-Temperature Oxidation Reaction)] AXO_{3±δ-α} + αCO₂ → AXO_{3±δ} + αCO

In addition, the entire reaction is represented as follows.

[Entire Reaction] αCO₂ → αCO + (α/2)O₂

In addition, the two-step thermochemical cycle reaction, which uses the perovskite oxide as a catalyst for thermochemical methanol production as an example of the catalyst for the thermochemical fuel production, includes the following two steps of an oxygen releasing reaction and a mixed gas consisting of hydrogen and carbon monoxide generating reaction.

[Oxygen Releasing Reaction (High-Temperature Reduction Reaction)] AXO_{3±δ} → AXO_{3±δ-α} + (α/2)O₂

[Mixed Gas Generating Reaction (Low-Temperature Oxidation Reaction)] 2 AXO_{3±δ-α} + αH₂O + αCO₂ → 2 AXO_{3±δ} + αH₂ + αCO

In addition, the entire reaction is represented as follows.

[Entire Reaction] αH₂O + αCO₂ → αH₂ + αCO + (α/2)O₂

To accomplish the above-described object, the document provides the following means.
(1) A catalyst for thermochemical fuel production, which is used for producing the fuel from thermal energy by using a two-step thermochemical cycle of a first temperature and a second temperature that is equal to or lower than the first temperature, wherein the catalyst is formed from a perovskite oxide having a compositional formula of AXO_{3±δ} (provided that, 0≤δ<1). Here, A represents one or more of a rare-earth element, an alkaline earth metal element, and an alkali metal element, X represents one or more of a transition metal element and a metalloid element, and O represents oxygen.
(2) The catalyst for thermochemical fuel production according to (1), wherein the element A is one or more selected from a group consisting of La, Mg, Ca, Sr, and Ba, and the element X is one or more selected from a group consisting of Mn, Fe, Ti, Zr, Nb, Ta, Mo, W, Hf, V, Cr, Co, Ni, Cu, Zn, Mg, Al, Ga, In, C, Si, Ge, and Sn.
(3) The catalyst for producing thermochemical fuel production according to (2), wherein the element A is La, and the element X is Mn.
(4) The catalyst for thermochemical fuel production according to (3), wherein the element A is partially substituted with one or more of Sr, Ca, and Ba.
(5) The catalyst for thermochemical fuel production according to (3), wherein the element X is partially substituted with one or more of Fe, Ni, V, Cr, Sc, Ti, Co, Cu, and Zn.
(6) The catalyst for thermochemical fuel production according to (1), wherein the element A is La, the element X is Mn, La is partially substituted with Sr.
(7) The catalyst for thermochemical fuel production according to (6), wherein the substituted concentration (x; x represents an amount with an amount of La before substitution being set to 1) of Sr is 0.1 or more to less than 1.0.
(8) The catalyst for thermochemical fuel production according to (7), wherein Mn is partially substituted with Fe.
(9) The catalyst for thermochemical fuel production according to (8), wherein the substituted concentration (x; x represents an amount with an amount of Mn before substitution being set to 1) of Fe is 0.35 or more to 0.85 or less.
(10) The catalyst for thermochemical fuel production according to (1), wherein the element A is Ba, the element X is Ti, Ti is partially substituted with Mn.
(11) The catalyst for thermochemical fuel production according to (10), wherein the substituted concentration (x; x represents an amount with an amount of Ti before substitution being set to 1) of Mn is more than 0 to 0.5 or less.
(12) A method of producing fuel using thermochemical fuel production, wherein the catalyst for thermochemical fuel production according to any one of (1) to (11) is used.
(13) A method of producing fuel using thermochemical fuel production, which produces the fuel from thermal energy by using the catalyst for thermochemical fuel production according to any one of (1) to (11) and by using a two-step thermochemical cycle of a first temperature and a second temperature that is equal to or lower than the first temperature, wherein the first temperature is 600 to 1,600°C, and the second temperature is 400 to 1,600°C.
(14) The method of producing fuel using thermochemical fuel production according to (13), wherein the first temperature is attained by irradiation of condensed sunlight energy and heating, or by heating using waste heat.
(15) The invention provides a method of producing fuel using thermochemical fuel production, which produces the fuel from thermal energy by using a two-step thermochemical cycle of a first temperature and a second temperature that is equal to or lower than the first temperature, the method including: a process of heating a perovskite oxide having a compositional formula of AXO_{3±δ} (provided that, 0≤δ<1) to the first temperature to reduce the perovskite oxide; and a process of bringing a raw material gas into contact with the reduced perovskite oxide and oxidizing the perovskite oxide to produce the fuel.
(16) The method of producing fuel using thermochemical fuel production according to (15), wherein the fuel is hydrogen.
(17) The method of producing fuel using thermochemical fuel production according to (15), wherein the raw material gas includes water vapor.
(18) The document describes the method of producing fuel using thermochemical fuel production according to (15), wherein the raw material gas includes carbon dioxide and water vapor.

In addition, in this specification, the "thermochemical fuel production" is a concept broadly extending to a concept of "thermochemical hydrogen production" in which water is decomposed into oxygen and hydrogen in relatively mild thermal conditions by combination of a plurality of chemical reactions to fuels including hydrogen.

In addition, the case of "partially substituted with" is that in which the concentration (x) of the substituted element is in the range of more than 0 to less than 1 when the amount of the element to be substituted before substitution is set to 1.

In addition, even when the "second temperature" is the same as the "first temperature", thermochemical fuel production is possible by changing an atmosphere, but in a case where the atmosphere is the same in each case, the "second temperature" represents a temperature lower than the "first temperature".

In addition, in regard to "δ" in the "compositional formula AXO_{3±δ} (provided that, 0≤δ<1)", it is preferable that 0≤δ≤0.5, more preferably 0≤δ≤0.3, and still more preferably 0≤δ≤0.2.

### [Advantageous Effects of the Invention]

According to the document, it is possible to provide a catalyst, which is formed from a perovskite oxide, for thermochemical fuel production. According to the invention, it is possible to provide a method of producing fuel using thermochemical fuel production that is capable of allowing a fuel to be produced in a thermochemical manner.

The present document provides a new catalyst for thermochemical fuel production by using perovskite oxide AXO₃.

According to the invention, elements such as iron and manganese, which are, at present, abundantly found in the Earth's crust, are used, and thus the used amount of rare-earth elements may be reduced. Therefore, it is possible to provide a catalyst for thermochemical fuel production and a method of producing fuel using thermochemical fuel production, in which significant cost reduction may be anticipated, and this will enable the conversion of solar energy into chemical fuel in high efficiency and the storage thereof.

### [Brief Description of the Drawings]

FIG. 1 is a graph illustrating X-ray diffraction results of La_{0.8}Sr_{0.2}Mn₁₋ₓFeₓO_{3±δ} (x = 0 to 1).
FIG. 2 is secondary electron microscope photographs of La_{0.8}Sr_{0.2}Mn₁₋ₓFeₓO_{3±δ}·
FIG. 3 is a graph illustrating a hydrogen production amount in the case of using La_{0.8}Sr_{0.2}Mn₁₋ₓFeₓO_{3±δ} as a catalyst for thermochemical fuel (hydrogen) production.
FIG. 4 is a graph illustrating dependency of a hydrogen production (generation) amount on an iron concentration (x) in the case of using La_{0.8}Sr_{0.2}Mn₁₋ₓFeₓO_{3±δ} as a catalyst for thermochemical fuel (hydrogen) production.
FIG. 5 is a graph illustrating a cycle characteristic of the hydrogen production amount in the case of using La_{0.8}Sr_{0.2}Mn₁₋ₓFeₓO_{3±δ}·
FIG. 6(a) is a graph illustrating a cycle characteristic of the hydrogen production amount and an oxygen production amount in the case of using La_{0.8}Sr_{0.2}Mn₁₋ₓFeₓO_{3±δ}, and FIG. 6(b) is a graph illustrating a cycle characteristic of the hydrogen production amount and the oxygen production amount.
FIG. 7 is a graph illustrating a hydrogen production amount in the case of using (La_{0.8}Sr_{0.2})MnO_{3±δ}, (La_{0.8}Sr_{0.2})(Mn_{0.85}Ti_{0.15})O_{3±δ}, (La_{0.8}Sr_{0.2})(Mn_{0.85}Fe_{0.15})O_{3±δ}, (La_{0.8}Sr_{0.2})(Mn_{0.85}Ni_{0.15})O_{3±δ}, (La_{0.8}Sr_{0.2})(Mn_{0.85}Mg_{0.15})O_{3±δ}, and La(Mn_{0.5}Mg_{0.5})O_{3±δ} as a catalyst for thermochemical hydrogen production, respectively.
FIG. 8 is a graph illustrating a cycle characteristic of the hydrogen production amount in the case of using (La_{0.8}Sr_{0.2})MnO_{3±δ}, (La_{0.8}Sr_{0.2})(Mn_{0.85}Ti_{0.15})O_{3±δ}, (La_{0.8}Sr_{0.2})(Mn_{0.85}Fe_{0.15})O_{3±δ}, (La_{0.8}Sr_{0.2})(Mn_{0.85}Ni_{0.15})O_{3±δ}, and (La_{0.8}Sr_{0.2})(Mn_{0.85}Mg_{0.15})O_{3±δ} as a catalyst for producing thermochemical hydrogen, respectively.
FIG. 9 is a graph illustrating a hydrogen production amount and an oxygen production amount in a case where (La_{0.8}Sr_{0.2})CrO_{3±δ} as a catalyst for producing thermochemical hydrogen is used, a first temperature is set to 1,300°C, and a second temperature is set to 800°C.
FIG. 10 is a graph illustrating a hydrogen production amount and an oxygen production amount in a case where (La_{0.8}Sr_{0.2})CrO_{3±δ} as a catalyst for producing thermochemical hydrogen is used, a first temperature is set to 1,500°C, and a second temperature is set to 800°C.
FIG. 11 is a graph illustrating a hydrogen production amount in the case of using Ba(Ti_{0.6}Mn_{0.4})O_{3±δ} as a catalyst for thermochemical hydrogen production.
FIG. 12 is a graph illustrating a cycle characteristic of a hydrogen production amount in the case of using La₁₋ₓSrₓMnO_{3±δ} (x = 0, 0.1, 0.2) as a catalyst for thermochemical hydrogen production.
FIG. 13 is a graph illustrating a hydrogen production amount in the case of using La₁₋ₓSrₓMnO_{3±δ} (x = 0.1, 0.2, 0.3, 0.4, 0.5) as a catalyst for thermochemical hydrogen production.
FIG. 14 is a graph illustrating a hydrogen production amount in the case of using (La_{0.8}Sr_{0.2})Mn₁₋ₓAlₓO_{3±δ} (x = 0, 0.25, 0.5, 0.75) as a catalyst for thermochemical hydrogen production.
FIG. 15 is a graph illustrating a hydrogen production amount in the case of using (La_{0.8}Sr_{0.2})MnO_{3±δ} and (La_{0.8}Ba_{0.2})MnO_{3±δ} as a catalyst for thermochemical hydrogen production.
FIG. 16 is a graph illustrating a hydrogen production amount in the case of using (La_{0.8}Ba_{0.2})(Mn_{0.25}Fe_{0.75})O_{3±δ} as a catalyst for thermochemical hydrogen production.

### [Mode for Carrying Out the Invention]

Hereinafter, a catalyst for thermochemical fuel production and a method of producing fuel using thermochemical fuel production, to which the invention is applied, will be described with reference to the attached drawings. In addition, in the drawings that are used in the following description, characteristic portions are sometimes enlarged for easy understanding of the characteristics, and the ratio of dimensions or the like of each constituent element is not limited to an actual value. In addition, in the following description, exemplified examples are for illustrative purposes only.

The catalyst for thermochemical fuel production according to the document is a catalyst for thermochemical fuel production, which is used for producing the fuel from thermal energy by using a two-step thermochemical cycle of a first temperature and a second temperature that is equal to or lower than the first temperature. The catalyst is formed from a perovskite oxide having a compositional formula of AXO_{3±δ} (provided that, 0≤δ<1). Here, A represents one or more of a rare-earth element, an alkaline earth metal element, and an alkali metal element, X represents one or more of a transition metal element and a metalloid (semi-metallic) element, and O represents oxygen.

A value of δ may be determined in a range not deteriorating an effect of the invention.

Examples of the rare-earth element include Sc (scandium), Y (yttrium), La (lanthanum), Pr (praseodymium), Nd (neodymium), Pm (promethium), Sm (samarium), Eu (europium), Gd (gadolinium), Tb (terbium), Dy (dysprosium), Ho (holmium), Er (erbium), Tm (thulium), Yb (ytterbium), Lu (lutetium), and Ce (cerium).

Examples of the alkaline earth metal element include Be (beryllium), Mg (magnesium), Ca (calcium), Sr (strontium), Ba (barium), and Ra (radium).

Examples of the alkali metal include Li (lithium), Na (sodium), K (potassium), Rb (rubidium), Cs (cesium), and Fr (francium).

Examples of the transition metal element include first transition elements (3d transition elements) such as Sc (scandium), Ti (titanium), V( vanadium), Cr (chromium), Mn (manganese), Fe (iron), Co (cobalt), Ni (nickel), Cu (copper), and Zn (zinc), second transition elements (4d transition elements) such as Y (yttrium), Zr (zirconium), Nb (niobium), Mo (molybdenum), Tc (technetium), Ru (ruthenium), Rh (rhodium), Pd (palladium), Ag (silver), and Cd (cadmium), and third transition elements (4f transition elements) such as La (lanthanum), Pr (praseodymium), Nd (neodymium), Pm (promethium), Sm (samarium), Eu (europium), Gd (gadolinium), Tb (terbium), Dy (dysprosium), Ho (holmium), Er (erbium), Tm (thulium), Yb (ytterbium), Lu (lutetium), Hf (hafnium), Ta (tantalum), W (tungsten), Re (rhenium), Os (osmium), Ir (iridium), Pt (platinum), and Au (gold).

Examples of the metalloid element include B (boron), Si (silicon), Ge (germanium), As (arsenic), Sb (antimony), Te (tellurium), Se (selenium), Po (polonium), and At (astatine).

Examples of such compounds preferably include a catalyst in which the element A is La, and the element X is Mn; a catalyst in which the element A is La, the element X is Mn, and La is partially substituted with one or more of Sr, Ca, and Ba; a catalyst in which the element A is La; the element X is Mn, La is partially substituted with one or more of Sr, Ca, and Ba and Mn is partially substituted with one or more of Fe, Ni, V, Cr, Sc, Ti, Co, Cu, and Zn; a catalyst in which the element A is Ba, the element X is Ti, and Ti is partially substituted with Mn; a catalyst in which the element A is one selected from a group consisting of Ca, Sr, and Ba, the element A is partially substituted with the substituted concentration of the element A being 0.01 or more to 0.5 or less; the element A is Ca or Sr, and the element X is Ti or Zr; and the like.

A method of producing fuel using thermochemical fuel production according to an embodiment of the document is a method of producing fuel using thermochemical fuel production, which produces the fuel from thermal energy by using the catalyst for thermochemical fuel production of the invention and by using a two-step thermochemical cycle of a first temperature and a second temperature that is equal to or lower than the first temperature. The first temperature is 600 to 1,600°C, and the second temperature is 400 to 1,600°C.

The first temperature and/or the second temperature are attained, for example, by irradiation of condensed sunlight energy and heating, or by heating using waste heat.

As the "waste heat", for example, waste heat of a power generator, a blast furnace, and the like may be used.

In the present invention, "two-step" means two different steps in which include two different conditions each other. Examples of "two-step" include a case in which the first step and the second step are conducted at different temperatures, and a case in which the first step and the second step are conducted at the same temperature, the first step is conducted with dry gas flow without water vapor and the second step is conducted with wet gas flow with water vapor.

In the case of producing hydrogen as a fuel, the first temperature may be set to 600 to 1,600°C (for example, 1,400°C), and the second temperature may be set to 400 to 1,600°C (for example, 800°C).

In the case of producing carbon monoxide as a fuel, the first temperature may be set to 600 to 1,600°C (for example, 1,400°C), and the second temperature may be set to 300 to 1,600°C (for example, 450°C).

In the case of producing a mixed gas consisting of hydrogen and carbon monoxide as a fuel, the first temperature may be set to 600 to 1,600°C (for example, 1,400°C), and the second temperature may be set to 300 to 1,600°C (for example, 800°C).

In the case of producing methane as a fuel, the first temperature may be set to 600 to 1,600°C (for example, 1,400°C), and the second temperature may be set to 300 to 1,600°C (for example, 450°C).

In the case of producing methanol as a fuel, the first temperature may be set to 600 to 1,600°C (for example, 1,400°C), and the second temperature may be set to 200 to 1,600°C (for example, 350°C).

A method of producing fuel using thermochemical fuel production according to the invention is a method of producing fuel using thermochemical fuel production, which produces the fuel from thermal energy by using a two-step thermochemical cycle of a first temperature and a second temperature that is lower than the first temperature. The method includes a process of heating a perovskite oxide having a compositional formula of AXO_{3±δ} (provided that, 0≤δ<1) to the first temperature to reduce the perovskite oxide, and a process of bringing a raw material gas into contact with the reduced perovskite oxide and oxidizing the perovskite oxide to produce the fuel.

A value of δ may be determined in a range not deteriorating an effect of the invention.

The fuel, which is produced by the method of producing fuel using thermochemical fuel production of the invention is hydrogen.

As an example of the raw material gas, for example, water vapor is exemplified, but it is not limited thereto. Hydrogen is produced by using the water vapor. In addition, as other examples, carbon dioxide and water vapor may be exemplified. Methane or methanol may be produced by using carbon dioxide and water vapor.

First, an outline of a method of producing a catalyst for thermochemical hydrogen production, as an example of the catalyst for thermochemical fuel production of the invention, will be described.

In the production of the catalyst for thermochemical hydrogen, a known method of producing perovskite oxide may be used. For example, powders of raw materials (oxide, hydroxide, oxide-hydroxide, and the like) including elements of a desired perovskite oxide are weighed to obtain a target compositional ratio, and are mixed and crushed. Then, the resultant mixture is calcined, and then the resultant calcined material is fired to produce the catalyst for producing thermochemical hydrogen.

More specifically, an example of a method of producing La_{0.8}Sr_{0.2}Mn₁₋ₓFeₓO_{3±δ} will be described.

Porous pellet of La_{0.8}Sr_{0.2}Mn₁₋ₓFeₓO_{3±δ} were synthesized via solid-state reaction. First, raw material oxides (La₂O₃, SrCO₂, MnCO₃, and Fe₂O₃) were crushed using an attritor-milling and were calcined at 1,000°C for three hours in air. These powders, that were obtained, were put into a die with isopropanol and were sintered at 1,500°C for 6 hours to obtain the porous pellets.

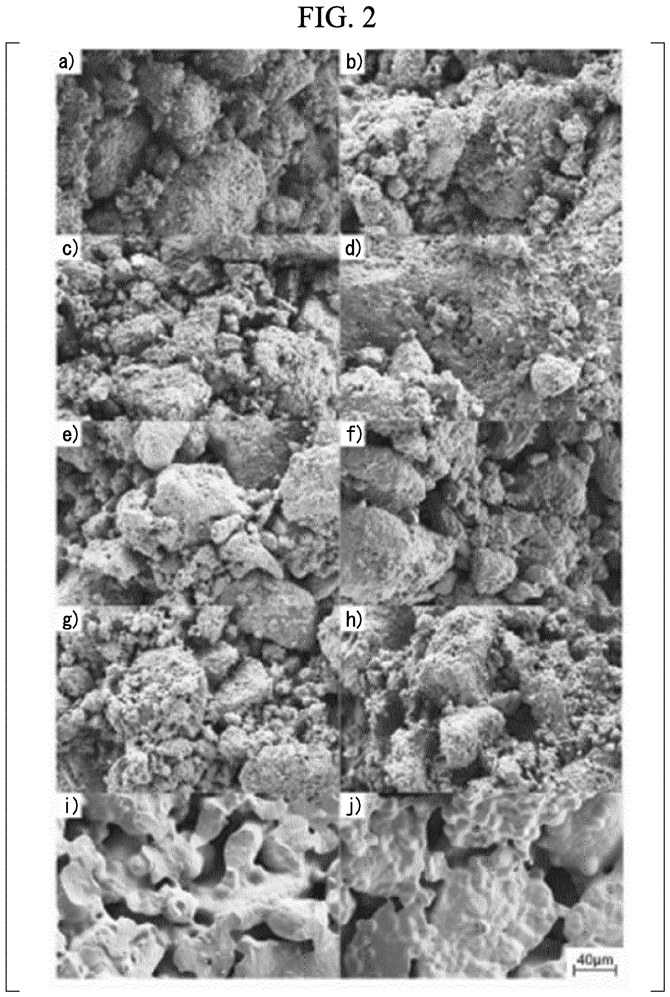

It was confirmed that the pellet of La_{0.8}Sr_{0.2}Mn₁₋ₓFeₓO_{3±δ} had a perovskite structure by X-ray diffraction (refer to FIG. 1). The resultant porosity of the pellet was approximately 60%. In addition, it was verified that the resultant pellets had various sizes of pores, ranging from a few to over 100 µm from a secondary electron microscopy image (refer to FIG. 2).

Next, an outline of production of hydrogen by using the catalyst for thermochemical hydrogen production that was obtained will be described.

For example, the production of hydrogen may be carried out as described below by using the catalyst for thermochemical hydrogen production.

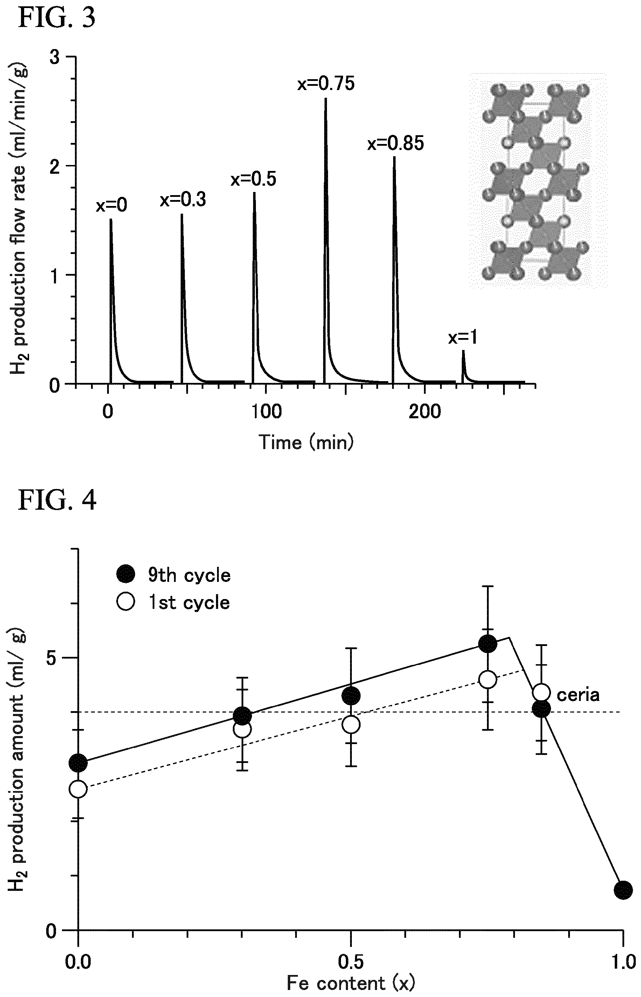

The porous pallet was placed inside an infrared furnace and was heated up to 1,400°C (corresponding to the "first temperature" of the two-step thermochemical cycle) in dry nitrogen containing 10 ppm of oxygen. At this time, it was observed that oxygen was released from the pellet by using mass spectroscopy. Then, the pellet was cooled down to 800°C (corresponding to the "second temperature" of the two-step thermochemical cycle), followed by flowing 10% water vapor containing argon gas. In the case of x=0, hydrogen evolution was observed at 800°C with an amount of 3 ml/g (corresponding to 60% or less of a hydrogen evolution amount in the case of using undoped ceria). The hydrogen evolution reaction was completed within 10 minutes as shown in FIG. 3.

The hydrogen evolution was reproducible over 9 cycles with slight increase in the amount, as shown in FIG. 4.

It seems the catalytic center of water oxidation in photosynthesis is a site of oxygen atom which is bonded to a metal atom with a longer bond length. La_{0.8}Sr_{0.2}Mn₁₋ₓFeₓO_{3±δ}, in which manganese is partially replaced with iron and which is a candidate to obtain a longer metal-oxygen bond length in the perovskite, could have alonger metal-oxygen bond length. Analysis results of La_{0.8}Sr_{0.2}Mn₁₋ₓFeₓO_{3±δ} are shown below.

FIG. 1 shows a graph illustrating X-ray diffraction results of La_{0.8}Sr_{0.2}Mn₁₋ₓFeₓO_{3±δ}. Horizontal axis shows diffraction angle (degree) and vertical axis shows diffraction intensity (arbitariry unit). X represents a concentration of Fe (that is an amount when an amount of Mn before substitution is set to 1), and X-ray diffraction results are shown in the case of x=0 (corresponding to a case of not containing Fe), in the case of x=0.3 (30 at%), in the case of x=0.5 (50 at%), in the case of x=0.75 (75 at%), and in the case of x=1 (100 at%; a case in which Mn atoms are entirely substituted with Fe atoms). As shown in FIG. 1, the perovskite structure is maintained at the entire iron concentrations (x) of La_{0.8}Sr_{0.2}Mn₁₋ₓFeₓO_{3±δ}. Differential scanning calorimetry also showed no evidence in phase transformation up to 1,400°C.

In addition, bold lines represent results before a thermal cycle and narrow lines represent results after the thermal cycle, and it can be seen that all of these show a perovskite structure.

FIG. 2 shows secondary electron microscope photographs of La_{0.8}Sr_{0.2}Mn₁₋ₓFeₓO_{3±δ}. In the drawing, (a), (c), (e), (g), and (i) represent the secondary electron microscope photographs before the thermal cycle, and (b), (d), (f), (h), and (j) represent the secondary electron microscope photographs after the thermal cycle of 800 to 1400°C. In (a) and (b), the iron concentration x is 0, in (c) and (d), the iron concentration x is 0.3 (not according to the invention), in (e) and (f), the iron concentration x is 0.5, in (g) and (h), the iron concentration x is 0.75, and in (i) and (j), the iron concentration x is 1 (not according to the invention). The size of the bar scale is 40 µm, as shown at the bottom right of Fig. 2.

It can be seen that the porous structure is maintained after the thermal cycle in any specimen.

FIG. 3 shows a graph illustrating a hydrogen production amount in the case of using La_{0.8}Sr_{0.2}Mn₁₋ₓFeₓO_{3±δ} as a catalyst for thermochemical hydrogen production. Horizontal axis shows time (minute) and vertical axis shows hydrogen production flow rate per unit gram (ml/min/g). The first temperature and the second temperature of the thermal (thermochemical) cycle were 1,400°C and 800°C, respectively.

As shown in FIG. 3, as the content of Fe increased in the order of the case in which the concentration (x) of iron (Fe) was 0 (corresponding to the case of not containing Fe), the case of x=0.3 (30 at%), the case according to the invention of x=0.5 (50 at%), and the case of x=0.75 (75 at%), the hydrogen production amount increased, and the hydrogen production amount in the case in which x is 0.75 was approximately 1.6 times the case of not containing Fe (x=0). The hydrogen production amount in the case of x=0.85 (85 at%) decreased by 15% compared to the case of x=0.75. In a case in which all of the Mn atoms were substituted with Fe atoms (x=1), the hydrogen production amount was approximately 10% of that of the case of x=0.75.

In the case of using La_{0.6}Sr_{0.4}MnO_{3±δ} as a catalyst for thermochemical hydrogen production (not according to the invention), in which the first temperature and the second temperature of the thermal (thermochemical) cycle were 1,400°C and 800°C, respectively, the hydrogen production amount was 7.5 ml/g.

FIG. 4 shows a graph illustrating dependency of a hydrogen production (generation) amount on an iron concentration (x) in the case of using La_{0.8}Sr_{0.2}Mn₁₋ₓFeₓO_{3±δ} as a catalyst for thermochemical hydrogen production. Horizontal axis shows iron concentration and vertical axis shows hydrogen production amount per unit gram (ml/g). The first temperature and the second temperature of the thermal cycle were 1,400°C and 800°C, respectively.

Results indicated by a white circle represent first cycle results, and results indicated by a black circle represent ninth cycle results.

As shown in FIG. 4, the hydrogen production amount in a case where x is 0.35 to 0.85 was larger than a hydrogen production amount (4.0 mg/l) of ceria, and the hydrogen production amount in the case of x=0.75 was 5.3 mg/l and was larger than the hydrogen production amount of ceria by 30% or more.

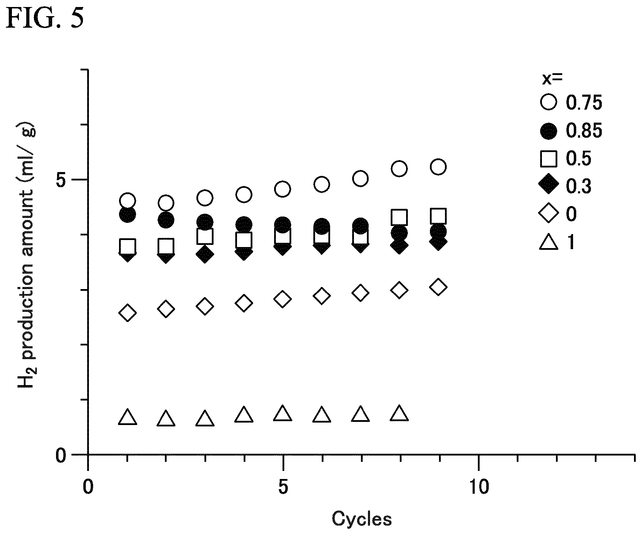

FIG. 5 shows a graph illustrating a cycle characteristic of the hydrogen production amount in the case of using La_{0.8}Sr_{0.2}Mn₁₋ₓFeₓO_{3±δ} as the catalyst for thermochemical hydrogen production. Horizontal axis shows cycles and vertical axis shows hydrogen production amount per unit gram (ml/g). The first temperature and the second temperature of the thermal cycle were 1,400°C and 800°C, respectively.

In a case where the iron concentration x is 0, 0.3, 0.5, 0.75, and 1, the hydrogen production amount gradually increased with the cycle. In a case of the iron concentration x=0.85, the hydrogen production amount gradually decreased with the cycle. In addition, although not shown in FIG. 5, the hydrogen production amount became constant at fifteen cycles.

A cycle characteristic, which is stable in any case without being dependent upon the iron concentration, was obtained.

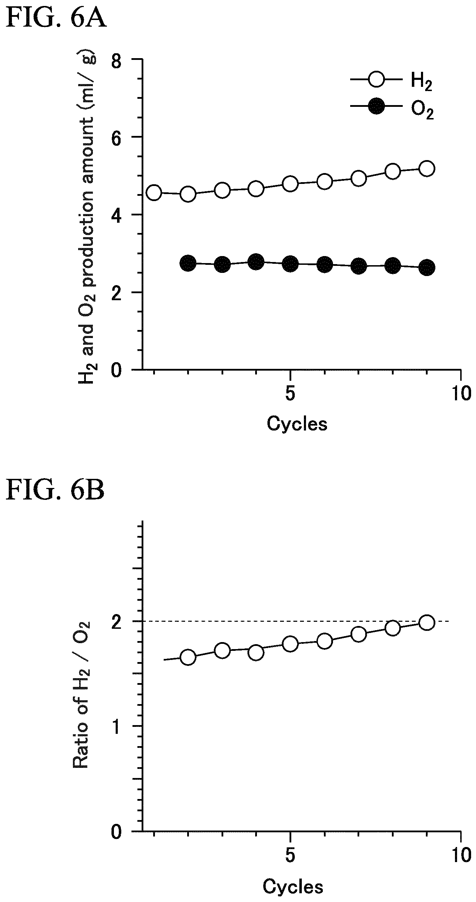

FIG. 6(a) shows a graph illustrating a cycle characteristic of the hydrogen production amount and the oxygen production amount in the case of using La_{0.8}Sr_{0.2}Mn₁₋ₓFeₓO_{3±δ}. Horizontal axis shows cycles and vertical axis shows hydrogen and oxygen production amount per unit gram (ml/g). FIG. 6(b) shows a graph illustrating a cycle characteristic of the hydrogen production amount and the oxygen production amount. Horizontal axis shows cycles and vertical axis shows ratio of hydrogen production amount for oxygen production amount. The first temperature and the second temperature of the thermal cycle were 1,400°C and 800°C, respectively.

As shown in FIG. 6(b), a ratio (H₂ amount/O₂ amount) of the hydrogen production amount and the oxygen production amount is approximately two, and FIG. 6(b) shows results of water splitting.

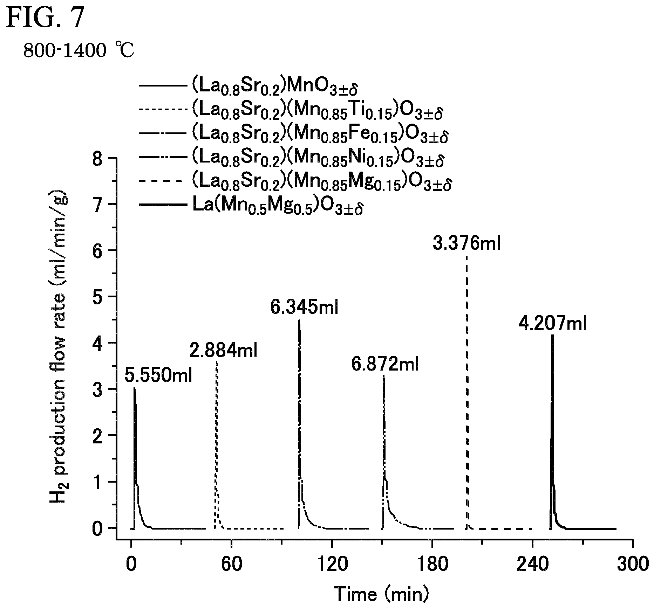

FIG. 7 shows a graph illustrating a hydrogen production amount in the case of using (La_{0.8}Sr_{0.2})MnO_{3±δ}, (La_{0.8}Sr_{0.2})(Mn_{0.85}Ti_{0.15})O_{3±δ}, (La_{0.8}Sr_{0.2})(Mn_{0.85}Fe_{0.15})O_{3±δ}, (La_{0.8}Sr_{0.2})(Mn_{0.85}Ni_{0.15})O_{3±δ}, (La_{0.8}Sr_{0.2})(Mn_{0.85}Mg_{0.15})O_{3±δ}, and La(Mn_{0.5}Mg_{0.5})O_{3±δ} as a catalyst for thermochemical hydrogen production. The hydrogen production amount is indicated by a flow rate (ml/min/g) per unit gram. Horizontal axis shows time (minute) and vertical axis shows hydrogen production flow rate per unit gram (ml/min/g). The first temperature and the second temperature of the thermal (thermochemical) cycle were 1,400°C and 800°C.

As shown in FIG. 7, even when perovskite oxide in which an A site is allocated to La, or La and Sr, and an X site is allocated to Mn, or Mn and at least one of Ti, Fe, Ni, and Mg is used in the catalyst for thermochemical hydrogen production, hydrogen may be produced in a thermochemical manner.

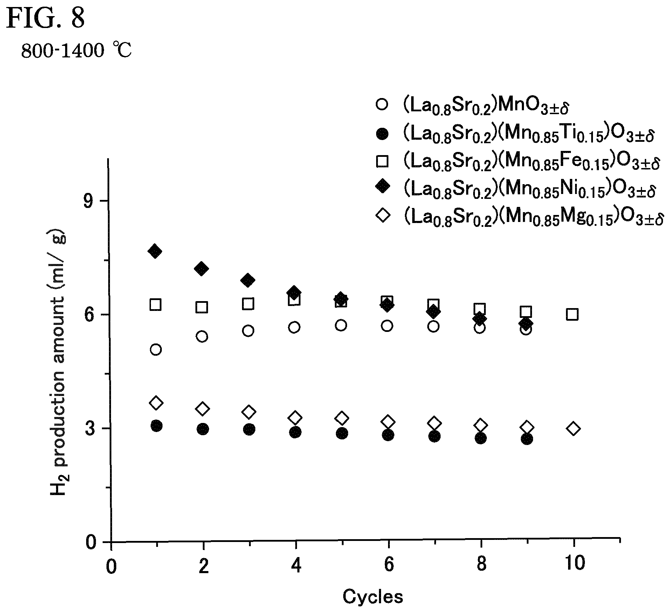

FIG. 8 shows a graph illustrating a cycle characteristic of the hydrogen production amount in the case of using (La_{0.8}Sr_{0.2})MnO_{3±δ}, (La_{0.8}Sr_{0.2})(Mn_{0.85}Ti_{0.15})O_{3±δ}, (La_{0.8}Sr_{0.2})(Mn_{0.85}Fe_{0.15})O_{3±δ}, (La_{0.8}Sr_{0.2})(Mn_{0.85}Ni_{0.15})O_{3±δ}, and (La_{0.8}Sr_{0.2})(Mn_{0.85}Mg_{0.15})O_{3±δ} as a catalyst for thermochemical hydrogen production. Horizontal axis shows cycles and vertical axis shows hydrogen production amount per unit gram (ml/g). The first temperature and the second temperature of the thermal cycle were 1,400°C and 800°C, respectively.

In the case of using (La_{0.8}Sr_{0.2})MnO_{3±δ} as the catalyst for thermochemical hydrogen production, the hydrogen production amount gradually increased with the cycle and became constant at approximately the fourth cycle.

In the case of using (La_{0.8}Sr_{0.2})(Mn_{0.85}Ti_{0.15})O_{3±δ} as the catalyst for thermochemical hydrogen production, the hydrogen production amount gradually decreased with the cycle.

In the case of using (La_{0.8}Sr_{0.2})(Mn_{0.85}Fe_{0.15})O_{3±δ} as the catalyst for thermochemical hydrogen production, the hydrogen production amount gradually decreased with the cycle.

In the case of using (La_{0.8}Sr_{0.2})(Mn_{0.85}Ni_{0.15})O_{3±δ} as the catalyst for thermochemical hydrogen production, the hydrogen production amount gradually decreased with the cycle. At the ninth cycle, the hydrogen production amount became substantially the same as the case of using (La_{0.8}Sr_{0.2})MnO_{3±δ}.

In the case of using (La_{0.8}Sr_{0.2})(Mn_{0.85}Mg_{0.15})O_{3±δ} as the catalyst for thermochemical hydrogen production, the hydrogen production amount gradually decreased with the cycle.

In the case of using (La_{0.8}Sr_{0.2})(Mn_{0.85}Ti_{0.15})O_{3±δ}, and (La_{0.8}Sr_{0.2})(Mn_{0.85}Mg_{0.15})O_{3±δ}, the hydrogen production amount was approximately 3 ml/g.

In the case of using (La_{0.8}Sr_{0.2})MnO_{3±δ}, (La_{0.8}Sr_{0.2})(Mn_{0.85}Fe_{0.15})O_{3±δ}, and (La_{0.8}Sr_{0.2})(Mn_{0.85}Ni_{0.15})O_{3±δ}, the hydrogen production amounts at the first cycle were approximately 5 ml/g, 6 ml/g, and 7 ml/g, respectively, but approaching the tenth cycle, all of the hydrogen production amounts became approximately 6 ml/g.

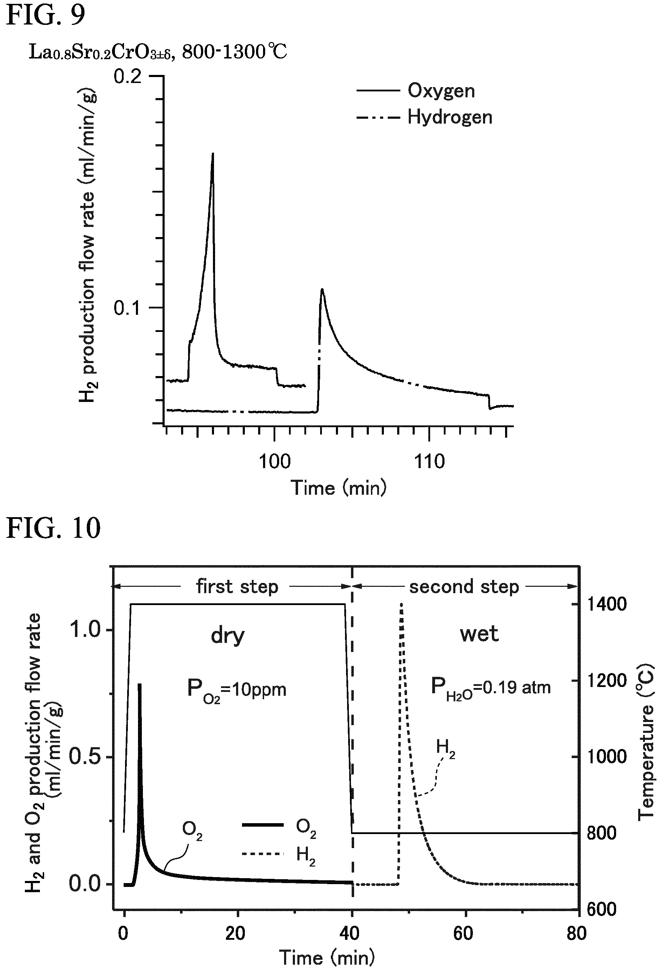

FIG. 9 shows a graph illustrating a hydrogen production amount and an oxygen production amount in a case of using (La_{0.8}Sr_{0.2})CrO_{3±δ} as the catalyst for thermochemical hydrogen production. The production amount is indicated by a flow rate (ml/min/g) per unit gram. Horizontal axis shows time (minute) and vertical axis shows hydrogen production flow rate per unit gram (ml/min/g). Solid line shows oxygen production flow rate unit gram and two-dot chain line shows hydrogen production flow rate per unit gram. The first temperature and the second temperature of the thermal (thermochemical) cycle were 1,300°C and 800°C, respectively.

As shown in FIG. 9, hydrogen could be thermochemically produced in the case of using (La_{0.8}Sr_{0.2})CrO_{3±δ} as the catalyst for thermochemical hydrogen production.

FIG. 10 shows a graph illustrating a hydrogen production amount and an oxygen production amount in the case of using (La_{0.8}Sr_{0.2})CrO_{3±δ} as the catalyst for producing thermochemical hydrogen similarly to FIG. 9. The first temperature and the second temperature of the thermal (thermochemical) cycle were 1,400°C and 800°C, respectively. Horizontal axis shows time (minute) and vertical axis shows hydrogen production flow rate unit gram (ml/min/g) or oxygen production flow rate per unit gram (ml/min/g). Solid line shows oxygen production flow rate per unit gram and dotted line shows hydrogen production flow rate per unit gram. The right vertical axis shows temperature (°C). FIG. 10 shows that the first step (Oxygen Releasing Reaction (High-Temperature Reduction Reaction) was performed at 1,400 °C for 40 minutes, then the second step (Hydrogen Generating Reaction (Low-Temperature Oxidation Reaction)) was performed at 800 °C.

As shown in FIG. 10, hydrogen could be thermochemically produced at high ratio of hydrogen production amount for oxygen production amount in the case of using (La_{0.8}Sr_{0.2})CrO_{3±δ} as the catalyst for thermochemical hydrogen production.

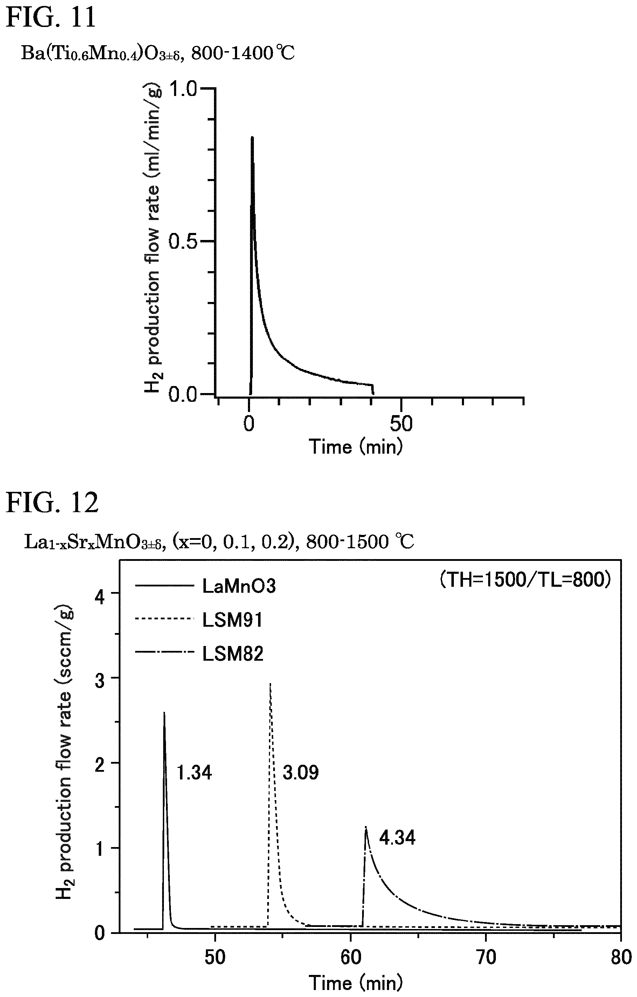

FIG. 11 shows a graph illustrating the hydrogen production amount in the case of using Ba(Ti_{0.6}Mn_{0.4})O_{3±δ} as the catalyst for thermochemical hydrogen production. Horizontal axis shows time (min) and vertical axis shows hydrogen production amount per unit gram (ml/g). The first temperature and the second temperature of the thermal (thermochemical) cycle were 1,400°C and 800°C, respectively.

As shown in FIG. 11, even when perovskite oxide in which an A site is allocated to Ba, and an X site is allocated to Mn and Ti is used in the catalyst for producing thermochemical hydrogen, hydrogen could be produced in a thermochemical manner.

FIG. 12 shows a graph illustrating a cycle characteristic of the hydrogen production amount in the case of using La₁₋ₓSrₓMnO_{3±δ} (x = 0, 0.1, 0.2) as the catalyst for producing thermochemical hydrogen. The production amount is indicated by a flow rate (sccm/g) per unit gram. Horizontal axis shows time (minute) and vertical axis shows hydrogen production flow rate per unit gram (sccm/g). Solid line (LaMnO3) shows hydrogen production flow rate unit gram in the case of x=0. Dotted line (LSM91) shows hydrogen production flow rate per unit gram in the case of x=0.1. One-dotted chain line, (LSM92) shows hydrogen production flow rate per unit gram in the case of x=0.2. The first temperature and the second temperature of the thermal (thermochemical) cycle were 1,500°C and 800°C, respectively.

The higher the concentration of Sr was, the further the hydrogen production amount increased, and the hydrogen production amount in the case of x=0.2 was 3 times that in the case of x=0.

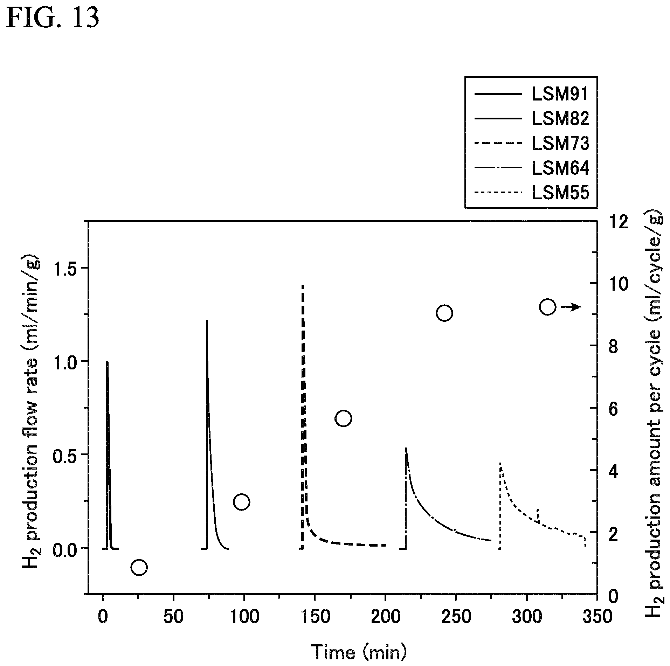

FIG. 13 shows a graph illustrating a cycle characteristic of the hydrogen production amount in the case of using La₁₋ₓSrₓMnO_{3±δ} (x = 0.1, 0.2, 0.3, 0.4, 0.5) as the catalyst for producing thermochemical hydrogen. The production amount for each case is indicated by a flow rate per unit gram (ml/min/g) at vertical axis at the left side and by a flow rate per cycle (ml/cycle/g) at vertical axis at the right side. Each line shown by LSM91, LSM82, LSM73, LSM64 and LSM55 in the figure shows a cycle characteristic of hydrogen production amount at x = 0.1, 0.2, 0.3, 0.4, 0.5, respectively, which is indicated by a flow rate per unit gram (ml/min/g). Each circle shown near each line shows a flow rate per cycle (ml/cycle/g) for each case. The first temperature and the second temperature of the thermal (thermochemical) cycle were 1,400°C and 800°C, respectively.

The higher the concentration of Sr was, the further the hydrogen production amount increased, and the hydrogen production amounts in the case of x=0.4 and x=0.5 were about 6 times that in the case of x=1.

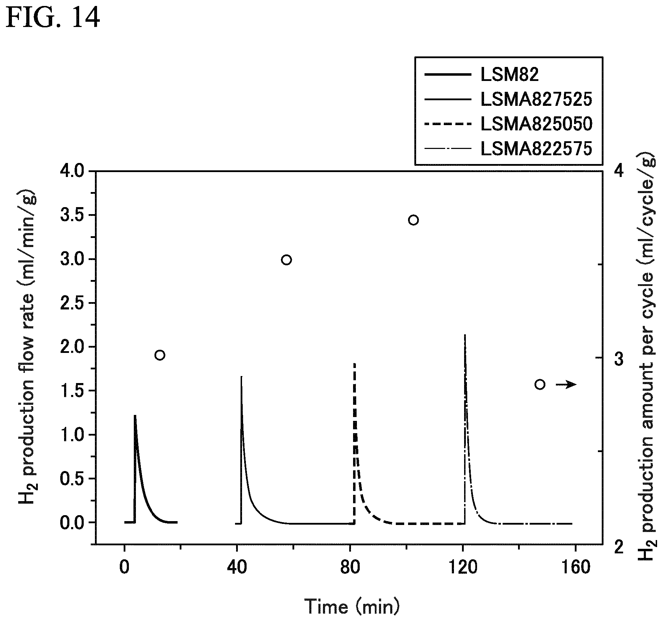

FIG. 14 shows a graph illustrating a cycle characteristic of the hydrogen production amount in the case of using La_{0.8}Sr_{0.2}Mn₁₋ₓAlₓO_{3±δ} (x =0, 0.25, 0.5, 0.75) as the catalyst for producing thermochemical hydrogen. The production amount for each case is indicated by a flow rate per unit gram (ml/min/g) at vertical axis at the left side and by a flow rate per cycle (ml/cycle/g) at vertical axis at the right side. Each line shown by LSM82, LSM827525, LSM825050 and LSM822575 in the figure shows a cycle characteristic of hydrogen production amount at x = 0, 0.25, 0.5, 0.75, respectively, which is indicated by a flow rate per unit gram (ml/min/g). Each circle shown near each line shows a flow rate per cycle (ml/cycle/g) for each case. The first temperature and the second temperature of the thermal (thermochemical) cycle were 1,400°C and 800°C, respectively.

The hydrogen production amount gradually increased until Al concentration became the same concentration as that of Mn concentration since zero. The hydrogen production amount decreased when Al concentration became higher (x=0.75).

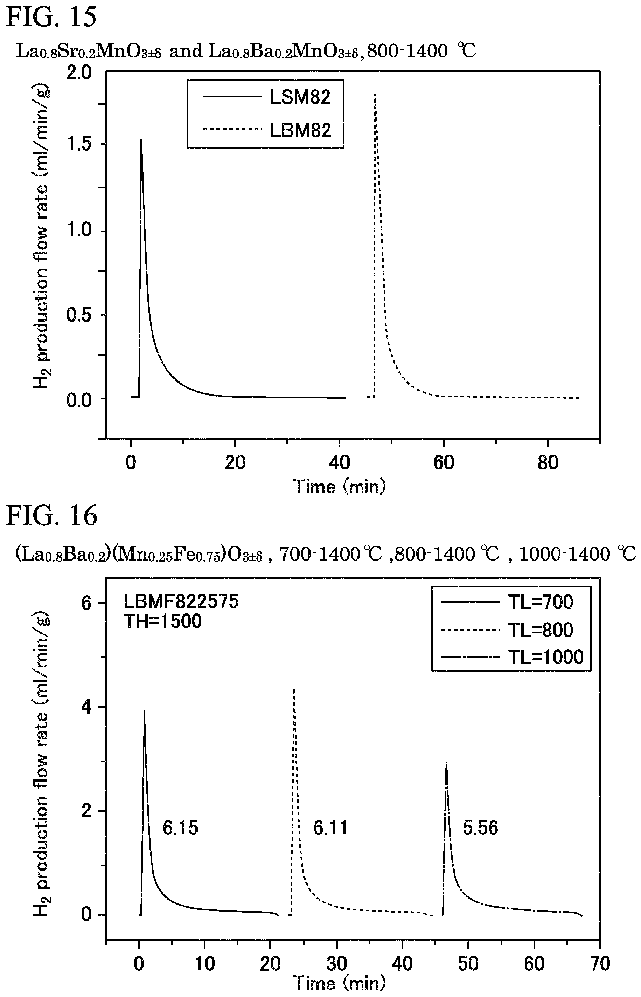

FIG. 15 shows a graph illustrating a hydrogen production amount in the case of using (La_{0.8}Sr_{0.2})MnO_{3±δ} and (La_{0.8}Ba_{0.2})MnO_{3±δ} as a catalyst for thermochemical hydrogen production. The production amount is indicated by a flow rate (ml/min/g) per unit gram. Horizontal axis shows time (minute) and vertical axis shows hydrogen production flow rate per unit gram (ml/min/g). Solid line (LSM82) shows hydrogen production flow rate of (La_{0.8}Sr_{0.2})MnO_{3±δ} and dotted line (LBM82) shows hydrogen production flow rate of (La_{0.8}Ba_{0.2})MnO_{3±δ}. The first temperature and the second temperature of the thermal (thermochemical) cycle were 1,400°C and 800°C, respectively.

Even when the perovskite oxide in which Sr of (La_{0.8}Sr_{0.2})MnO_{3±δ} was substituted with Ba was used in the catalyst for thermochemical hydrogen production, the hydrogen production amount was not changed too much.

FIG. 16 is a graph illustrating the hydrogen production amount in the case of using (La_{0.8}Ba_{0.2})(Mn_{0.25}Fe_{0.75})O_{3±δ} as the catalyst for thermochemical hydrogen production. The production amount is indicated by a flow rate (ml/min/g) per unit gram. Horizontal axis shows time (minute) and vertical axis shows hydrogen production flow rate per unit gram (ml/min/g). The first temperature of the thermal (thermochemical) cycle was 1,400°C, but the second temperatures thereof were 700°C (solid line), 800°C (dotted line), and 1,000°C (two-dot chain line), respectively.

In the case where the second temperatures were 700°C and 800°C, the hydrogen production amount did not vary largely. However, conversely, in the case where the second temperature was 1,000°C, the hydrogen production amount decreased compared to the case in which the second temperatures were 700°C and 800°C by approximately 10%.

In the solar thermochemical hydrogen production, the concentrated solar energy needs to be absorbed by the perovskite oxide. The solar spectrum ranges from ultraviolet to visible and infrared region (250 nm to over 2700 nm). The absorbed photon excites electrons from the lower-state to the excited state, which will eventually be converted to heat via phonon. The solar absorbance measurement shows that the La_{0.8}Sr_{0.2}Mn_{0.25}Fe_{0.75}O_{3±δ} perovskite absorbs solar energy quite efficiently, at 4 times of ceria.

The elements composed in the perovskite are quite earth abundant. The earth abundance of iron and manganese are 35 and 0.6 times the earth abundance of carbon, respectively. Strontium (Sr) exists in the earth crust 5 times more than copper (Cu), and the lanthanum (La) is a half of copper.

In summary, the present inventors developed catalytic La_{0.8}Sr_{0.2}Mn₁₋ₓFeₓO_{3±δ} perovskites for thermochemical hydrogen production by mimicking the catalytic center of Mn₄CaO₅ cluster in photosystem II. Particularly, La_{0.8}Sr_{0.2}Mn_{0.25}Fe_{0.75}O_{3±δ} produces 5.3 ml/g of hydrogen in the thermochemical cycle between 800 and 1400°C. The advantages of utilizing nonstoichiometric perovskite over undoped ceria are more efficient solar absorbance by 4 times or less, the earth abundant element utilization for scalable solar fuel production, and lower temperature operation at 1400°C. The strontium which is abundant in the Earth in this system is completely soluble in lanthanum, and it is possible to mimic the rare-earth utilization in the catalytic perovskite.

### [Industrial Applicability]

The invention will allow the conversion of solar energy into chemical fuel in high efficiency and the storage thereof, and the obtained chemical fuel will be used as clean energy in the field of each industry and as clean industrial raw materials in chemical industry.

## Claims

1. A method of producing fuel using thermochemical fuel production,
which produces hydrogen as the fuel from thermal energy using a two-step thermochemical cycle of a first temperature and a second temperature that is equal to or lower than the first temperature, the method comprising:
a process of heating a perovskite oxide having a compositional formula of AXO_{3±δ} (provided that 0≤δ<1) to the first temperature to reduce the perovskite oxide; and,
a process of bringing water vapor that is raw material gas into contact with the reduced perovskite oxide and oxidizing the perovskite oxide to produce the fuel, at the second temperature,
**characterized in that**
the element A is La, the element X is Mn,
La is partially substituted with Sr, wherein the concentration of the Sr element is 0.2 and the concentration of La is 0.8; and
Mn is partially substituted with Fe, wherein the concentration x of the element Fe that is substituted is 0.35 or more to 0.85 or less, with x represents an amount of the element Fe with an amount of Mn before the substitution being set to 1, and
the first temperature is in the range of 600 to 1600°C, and
the second temperature is in the range of 400 to 1600°C.

## Patentansprüche

1. Verfahren zum Herstellen von Kraftstoff unter Verwendung von thermochemischer Kraftstoffherstellung,
welches Wasserstoff als den Kraftstoff aus thermischer Energie unter Verwendung eines zweistufigen thermochemischen Kreislaufs einer ersten Temperatur und einer zweiten Temperatur erzeugt, welche gleich oder niedriger als die erste Temperatur ist, wobei das Verfahren umfasst:
einen Vorgang des Erhitzens eines Perowskit-Oxids, welches eine Zusammensetzungsformel von AXO_{3±δ} aufweist (vorausgesetzt, dass 0≤δ<1), auf die erste Temperatur, um das Perowskit-Oxid zu reduzieren; und
einen Vorgang des Inkontaktbringens von Wasserdampf, welcher ein Ausgangsmaterialgas ist, mit dem reduzierten Perowskit-Oxid und Oxidierens des Perowskit-Oxids, um den Kraftstoff herzustellen, bei der zweiten Temperatur,
**dadurch gekennzeichnet, dass**
das Element A La ist, das Element X Mn ist,
La teilweise durch Sr substituiert ist, wobei die Konzentration des Elements Sr 0,2 beträgt und die Konzentration von La 0,8 beträgt; und
Mn teilweise durch Fe substituiert ist, wobei die Konzentration x des Elements Fe, welches substituiert ist, 0,35 oder mehr bis 0,85 oder weniger beträgt, wobei x eine Menge des Elements Fe repräsentiert, wobei die Menge des Mn vor der Substitution auf 1 gesetzt wird, und
die erste Temperatur in dem Bereich von 600 bis 1600 °C liegt, und
die zweite Temperatur in dem Bereich von 400 bis 1600 °C liegt.

## Revendications

1. Procédé de production de combustible utilisant une production de combustible thermochimique,
qui produit de l'hydrogène en tant que combustible à partir d'énergie thermique en utilisant un cycle thermochimique en deux étapes constitué d'une première température et d'une seconde température qui est égale ou inférieure à la première température, le procédé comprenant :
un processus de chauffage d'un oxyde de pérovskite ayant une formule de composition d'AXO_{3±δ} (à condition que 0≤δ<l) jusqu'à la première température pour réduire l'oxyde de pérovskite ; et,
un processus de mise en contact de vapeur d'eau qui est un gaz de matière première avec l'oxyde de pérovskite réduit et d'oxydation de l'oxyde de pérovskite pour produire le combustible, à la seconde température,
**caractérisé en ce que**
l'élément A est La, l'élément X est Mn,
La est partiellement substitué par Sr, dans lequel la concentration de l'élément Sr est de 0,2 et la concentration de La est de 0,8 ; et
Mn est partiellement substitué par Fe, dans lequel la concentration x de l'élément Fe qui est substitué est de 0,35 ou plus à 0,85 ou moins, x représentant une quantité de l'élément Fe avec une quantité de Mn avant que la substitution ne soit établie à 1, et
la première température est dans la plage de 600 à 1 600 °C, et
la seconde température est dans la plage de 400 à 1 600 °C.
